(19) 

**Europäisches Patentamt**
**European Patent Office**
**Office européen des brevets**

(11) **EP 4 195 212 A1**

(12) **EUROPEAN PATENT APPLICATION**

(43) Date of publication:
**14.06.2023 Bulletin 2023/24**

(21) Application number: **21213167.6**

(22) Date of filing: **08.12.2021**

(51) International Patent Classification (IPC):
**G16H 10/40** (2018.01)    **G16H 50/20** (2018.01)

(52) Cooperative Patent Classification (CPC):
**G16H 10/40; G16H 50/20; G16H 50/30;**
**G16H 50/70**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **A3P Biomedical AB (publ)**
**111 35 Stockholm (SE)**

(72) Inventors:
• **ANDERSSON, Karl**
**755 78 Vänge (SE)**
• **WALLDEN, Mats**
**756 43 Uppsala (SE)**
• **MATTSON, Per**
**752 39 Uppsala (SE)**

(74) Representative: **AWA Sweden AB**
**Box 45086**
**104 30 Stockholm (SE)**

(54) **CERTAINTY-BASED MEDICAL CONCLUSION MODEL ADAPTATION**

(57)    Methods for providing medical conclusions and support therefore comprises measuring (S10) of quantities related to concentrations of at least three different biomarkers of control samples, sending (S12), receiving (S20) and storing (S22) the same in an archive memory. Stored data is retrieved (S26) as a response to a sending (S14) and receiving (S24) of a request for an adapted medical conclusion model and is processed (S28) into the certainty deduction model. The adapted medical conclusion model is processed (S30) based on the certainty deduction model, and is outputted (S32) and received (S17) and medical conclusions made from measurements of samples together with the control samples is provided (S19) based on the received adapted medical conclusion model. The certainty deduction model comprises a group model, determined for all control samples, and measurement entity performance characteristics, determined for measurements related to the first measurement entity that are performed less than a predetermined time ago.

Fig. 5A          Fig. 5B

EP 4 195 212 A1

**Description**

TECHNICAL FIELD

The present invention relates in general to evaluation of medical measurements, and in particular to systems and methods for certainty-based adapted medical conclusions and certainty-based medical conclusion model adaptation.

BACKGROUND

In most types of activities related to health and care, biochemical tests are today an important component. Biochemical measurements are commonly used as support for diagnoses, to monitor courses of health events, and to serve as a guide for treatment of diseases for a particular patient. A statistical treatment of a collection of biochemical measurements of a group of patients may also serve e.g. as a decision support for health care planning and medical resource provision.

Still, most biochemical assays provide single outputs, i.e. where the presence or concentration of one defined biomarker is measured. The use of multiplexed assays is, however, increasing. In a multiplexed assay, the presence or concentration of multiple biomarkers is determined at essentially the same time, e.g. same day or same week, in one or more aliquots of the same sample. This ensures that the different measurements are closely associated with each other. Each biomarker may be analyzed using the same device or multiple devices may be required to analyze the entire set of biomarkers. Resulting data is then combined to form an output, which may be used for a single risk estimate or a pattern of some sort indicative of sample characteristics. One example of a multiparametric test is EndoPredict, a breast cancer prognostic test. It analyses RNA expression of 8 target genes, 3 normalization genes, and 1 control gene. Obtained RNA expression levels are combined into a score, which is then combined with clinical features of the tumour (like tumour size). The score has been shown to predict the 10-year distant recurrence rate. Multiplexed assays typically increase efficiency but complicate the calibration procedure.

In general, measurement outputs have widely varying units, and varying linearity to response. This variability complicates evaluation of any biochemical output result, even for single output approaches. In cases where multiple assay results are to be combined, the situation grows even more complicated. If different laboratories are implementing the same assay using different types of analytical instruments, the inherent lab-to-lab, instrument-to-instrument, and instrument type-to-instrument type variation will add to the overall variation.

Laboratory quality control is a fundamental task in clinical laboratories where important medical statements or decisions are made based on measured values on sample specimens. One corner stone of practical laboratory quality control is the use of control samples. A sample specimen with known properties is repeatedly tested and the results are followed up to confirm or reject the test under study.

As discussed above, most laboratory tests are of singleton nature, i.e. one sample specimen is tested for one property which is then reported to the requesting entity. As an example, an elevated level of C-reactive protein (CRP) in a blood sample is considered to be indicative of inflammation. Accordingly, the vast majority of quality procedures for laboratories are designed for tests of singleton nature.

A common method to quality control a test of singular nature is to regularly test a control sample with known properties, and plot the results obtained from consecutive testing of the control sample in a Levey-Jennings plot. This type of control chart illustrates if obtained values for the control sample are constant. Should values for the control sample start to deviate in any manner, the measurement procedure should be investigated and corrected.

Another common method to quality control a test of singular nature is to regularly send a portion of a sample to another laboratory, ask the other laboratory to make the same test, and finally compare results. This procedure is known as proficiency testing.

As discussed above, multiparametric tests are emerging. However, quality control procedures tailored for the individual methods that together form a multiparametric test are however rare.

In a laboratory where procedures have been implemented to conduct a multiparametric method for estimating the risk of an individual to have a disease, there will be need for some type of quality control of each contributing measurement value. Assume that the multiparametric method is in part relying on the measurement of concentrations for a number of proteins. The laboratory would then typically be processing both samples from subjects for whom the risk of having disease is to be estimated, and control samples for the purpose of qualifying the measurement procedure as functional. Control samples could for example be measured once per day or once per measurement batch, the latter where the measurement procedure would run batches of approximately 30-100 samples.

The staff at the laboratory would then face the challenge of determining if the measurement procedure is sufficiently functional in order to produce a high-quality output from a predefined multiparametric test. Furthermore, the staff would also be interested in obtaining some knowledge about the effect of typical variations of input data on the estimated multiparametric risk. In other words, could normal measurement variations or inaccuracies result in another multiparametric test verdict.

One tentative solution to this problem is to apply quality procedures that resembles the procedures used for singleton cases. Although a singleton procedure may work, the application on multiparametric cases may be unnecessarily strict and may require an excess of work. Multiparametric tests are often such that larger errors on the input data is acceptable, because all results are combined in an algorithmic manner and deviations on input data will statistically cancel in the process of combining the multiple parameters into a single output statement. Multiparametric tests are in other words typically more tolerant to errors in single measurements. Singleton quality control procedures completely disregard this aspect.

The same principle of using singleton quality procedures seen from a different angle concerns pass rate. As an example, if singleton quality control is configured with limits that pass 99% of the data, a multiparametric procedure relying on 5 measurements would result in a pass rate of $0.99^5$, i.e. approximately 95% pass rate. Similarly, a multiparametric procedure relying on 20 measurements would, with the same pass criteria for each individual procedure, have approximately 82% pass rate.

There is therefore a need for a multiparametric approach for medical conclusions, tailored to match the multiparametric use of the input parameters.

SUMMARY

A general object is to provide improved support for medical conclusions based on multiparametric tests.

The above object is achieved by methods and devices according to the independent claims. Preferred embodiments are defined in dependent claims.

In general words, in a first aspect, a system for support in medical conclusions comprises a processing system, having at least one processor and an archive memory. The system for support in medical conclusions further comprises an input configured for receiving multiple items of measured quantities related to concentrations of at least three different biomarkers of at least two control samples, sample identity of the at least two control samples and measurement entity identification data of the measuring entity that has performed the measurements. The processing system is configured for storing the received measure quantities, the sample identity and measurement entity identification data in the archive memory. The input is further configured for receiving a request for an adapted medical conclusion model associated with a first measurement entity from a requesting party. The processing system is further configured for retrieving stored data from the archive memory. The processing system is further configured for processing the retrieved stored data into a certainty deduction model associated with the first measurement entity. The certainty deduction model comprises a group model and measurement entity performance characteristics. The group model is determined on stored data from the archive for all control samples related to a predetermined set of multiple measurement entities. The measurement entity performance characteristics is determined on stored data from the archive for measurements of control samples related to the first measurement entity that are performed less than a predetermined time ago. The processing system is further configured for processing the retrieved stored data into the adapted medical conclusion model, adapted to the certainty deduction model associated with the first measurement entity. The system for support in medical conclusions further comprises an output configured for outputting the adapted medical conclusion model associated with the first measurement entity to the requesting party.

In a second aspect, a system for medical conclusions comprises a measurement entity for measuring of quantities related to concentrations of at least three different biomarkers of samples. The samples comprise a plurality of samples associated with individuals and at least two control samples. The system for medical conclusions further comprises an output configured for sending measured quantities related to concentrations of at least three different biomarkers of the at least two control samples, sample identity of the at least two control samples, and measurement entity identification to a system for support in medical conclusions. The output is further configured for sending a request for an adapted medical conclusion model associated with the measurement entity to the system for support in medical conclusions. The system for medical conclusions further comprises an input configured for receiving the adapted medical conclusion model associated with the measurement entity from the system for support in medical conclusions. The adapted medical conclusion model is adapted based on a certainty deduction model comprising a group model and measurement entity performance characteristics. The group model is determined on stored data for control samples related to a predetermined set of multiple measurement entities. The measurement entity performance characteristics is determined on stored data for measurements of control samples related to the measurement entity that are performed less than a predetermined time ago. The system for medical conclusions further comprises a processing unit configured for deducing medical conclusions from measurements of the samples associated with individuals, made together with the at least two control samples, by use of the received adapted medical conclusion model.

In a third aspect, a method for providing support in medical conclusions, comprising receiving of multiple items of measured quantities related to concentrations of at least three different biomarkers of at least two control samples, sample identity of the at least two control samples, and measurement entity identification data of the measuring entity that has performed the measurements. The received measure quantities, the sample identity and measurement entity identification data are stored in an archive memory. A request for an adapted medical conclusion model associated with a first measurement entity is received from a requesting party. Stored data is retrieved from the archive memory. The retrieved stored data is processed in a processing system into a certainty deduction model associated with the first measurement entity. The certainty deduction model comprises a group model and measurement entity performance characteristics. The group model is determined on stored data from the archive memory for all control samples related to a predetermined set of multiple measurement entities. The measurement entity performance characteristics is determined on stored data from the archive memory for measurements of control samples related to the first measurement entity that are performed less than a predetermined time ago. The retrieved stored data is processed into the adapted medical conclusion model, adapted to the certainty deduction model associated with the first measurement entity. The adapted medical conclusion model associated with the first measurement entity is outputted to the requesting party.

In a fourth aspect, a method for providing non-diagnostic medical conclusions comprises measuring of quantities related to concentrations of at least three different biomarkers of samples. The samples comprise a plurality of samples associated with individuals and at least two control samples. Measured quantities related to concentrations of at least three different biomarkers of the at least two control samples, sample identity of the at least two control samples, and measurement entity identification are sent to a system for support in medical conclusions. A request for an adapted medical conclusion model associated with the measurement entity is sent to the system for support in medical conclusions. The adapted medical conclusion model associated with the measurement entity is received from the system for support in medical conclusions. The adapted medical conclusion model is adapted based on a certainty deduction model comprising a group model and measurement entity performance characteristics. The group model is determined on stored data from the archive for all control samples related to a predetermined set of multiple measurement entities. The measurement entity performance characteristics is determined on stored data from the archive for measurements of control samples related to the measurement entity that are performed less than a predetermined time ago. Non-diagnostic medical conclusions are deducing from measurements of the samples associated with individuals, made together with the at least two control samples, based on the received adapted medical conclusion model.

One advantage with the proposed technology is that medical conclusions may be taken with better accuracy. Other advantages will be appreciated when reading the detailed description.

## BRIEF DESCRIPTION OF THE DRAWINGS

The invention, together with further objects and advantages thereof, may best be understood by making reference to the following description taken together with the accompanying drawings, in which:

FIG. 1 illustrates schematically an embodiment of a two-level organization of systems associated with support for medical conclusions;
FIG. 2 illustrates schematically an embodiment of system for medical conclusions;
FIG. 3 illustrates schematically an embodiment of a system for medical conclusions;
FIG. 4 illustrates schematically an adapted medical conclusion model composition;
FIG. 5A is a flow diagram of steps of an embodiment of a method for providing medical conclusions;
FIG. 5B is a flow diagram of steps of an embodiment of a method for providing support in medical conclusions;
FIG. 6 is a flow diagram of steps of another embodiment of a method for providing support in medical conclusions; and
FIGS. 7A-D are diagrams of free PSA concentrations for two samples without and with batch adjustments.

## DETAILED DESCRIPTION

Throughout the drawings, the same reference numbers are used for similar or corresponding elements.

For the purpose of this disclosure and for clarity, the following definitions are made:

The term "biomarker" refers to a biological or biochemical signature which is confirmed or suspected to be related to a status of a living organism. A biomarker can for example be a protein present in blood, such as prostate specific antigen (PSA). A biomarker can also, as another example, be the allele setup for a particular nucleotide in the genome (such as a single nucleotide polymorphism).

The term "measurement procedure" refers to a procedure to estimate presence or concentration of a biomarker. As a non-limiting example, the concentration of a protein in blood can sometimes be quantified using a measurement procedure denoted Enzyme linked immunosorbent assay (ELISA), a well-known procedure which has been used several decades. As another non-limiting example, the presence of particular allele at a particular locus in the genome can be determined using a Taqman assay, a well-known procedure which has been used more than one decade.

The term "multiparametric test" refers to a test procedure carried out in a laboratory for the purpose of estimating the status of the tested object, where results from more than one measurement on one or more test specimens are combined into a composite value that is in turn related to the status. The tested object is commonly a mammal (most often a human), the status is often related to a health-related condition (such as diagnosis or prognosis of a disease), the test specimen is often a body fluid or tissue from the test object (such as a blood sample or saliva or urine from a mammal), and the measurement is often a measurement of presence or concentration of a biomarker.

The term "control sample" refers to a test specimen with known characteristics with respect to a particular biomarker. A control sample can be of synthetic or native origin. A synthetic control sample may be a composed of an aqueous preparation containing a known quantity of a recombinant protein. A native control sample may be composed of serum made from a blood donor, where the serum has been extensively characterized in terms of the quantity of a particular biomarker.

The term "medical conclusion" refers to a conclusion related to (i) health status or (ii) reactions to a health status. As nonlimiting examples, health status can be diagnosis of a disease (such as cancer) or it can be a condition such as pregnancy. A reaction to a health status can, as a nonlimiting example, be the conclusion that an individual with a disease no longer is in need for advanced care and therefore can be discharged from a hospital to recover at home. Another reaction to a health status can, as a nonlimiting example, be the conclusion that an individual is in need for a more careful examination by a different health care entity, such as primary care referring an individual to a urologist.

The term "Risk model" refers to a multiparametric algorithm that has more than one input and that estimates, directly or implicitly, the probability of a living organism having a health-related state. A risk model could for example combine inputs related to virus load in a living organism and output a probability of the individual being capable of transferring the virus to others.

The term "certainty deduction model" refers to a scheme that estimates the certainty or reliability or concordance of the output of a risk model. When applied for measurement values for an individual, a certainty deduction model typically has performance indicators related to the measurement procedure as well as risk model results as input and provides an output related to the reliability of the risk model result for the individual. When applied for the risk model per se, a certainty deduction model provides instructions for how to adapt the risk model to maintain reliability and concordance of the risk model in the hands of a particular laboratory.

The term "group model" refers to a generic type of certainty deduction model. A group model typically relies on average performance indicators acquired from multiple laboratories and hence represent a typical or average level of certainty that the multiparametric test in question would be able to provide. A group model can also contain and convey average performance indicator values originating from multiple laboratories to other entities that may need that information.

The term "measurement entity performance characteristics" refers to defined characteristics for measurement procedures. As a nonlimiting example, two suitable measurement entity performance characteristics for a measurement procedure related to a concentration determination are (i) lower limit of detection (i.e. the smallest concentration possible to measure) and (ii) reproducibility (i.e. how different repeated measurements on the same sample specimens is).

The term "adapted medical conclusion model" refers to a risk model that has been adapted to become useful in a particular setting, such as adapting a risk model to the performance characteristics of a particular measurement entity so that the risk model is functioning comparable to other measurement entities with their respective (different) performance characteristics.

The use of multiparametric tests have many advantages. The result is typically more precise than what is available by a series of separate single-parametric tests. Multiparametric tests are typically also less sensitive to individual measurement errors. The drawbacks are, however, that the evaluation of the multiparametric tests typically is so complex that it is essentially impossible for a laboratory staff or a physician to understand the connections between the individual measurement results and the final evaluation result. It is even more difficult for the laboratory staff or physician to assess the accuracy of the result, i.e. how certain is the outcome.

In most evaluation processes, a threshold value approach is used, which means that if a result exceeds a certain threshold (or falls below), an indication of a certain decease or other circumstance is declared. Further decisions and measures are then typically taken in an on/off manner, where subjects of all results falling below the threshold are grouped into one group and subjects of all results exceeding the threshold are grouped into another group. Any future treatment is then typically based solely on which group the subject belongs to. It is even with single-parametric tests unusual that the actual result value is considered, i.e. if the result is close to the threshold or far from the threshold. Even if a measurement result falls below the threshold, a result close to the threshold may have such inherent inaccuracies that there is a non-negligible probability that the true value in fact would have been exceeding the threshold. In such situations, further investigation may be helpful to further establish the actual conditions. However, as mentioned above, such considerations are rare, already using single-parametric tests, and when multiparametric tests are used, the complexity makes the possibilities to make such considerations very small.

When trying to analyze the properties of a multi-parametric model, in order to achieve some kind of accuracy information, one realizes that two main contributions may be identified. A first contribution is the modelling itself, i.e. what assumptions and modelling features are used and how they influence the result. All samples evaluated by the multi-parametric model will be influenced by such contributions. In general, the more reference measurements available, the better the evaluation of such group effects may be performed.

A second contribution to the accuracy analysis comes from the actual measurements. Different laboratories may e.g. implement the same assay using different types of analytical instruments, and the inherent lab-to-lab, instrument-to-instrument, and instrument type-to-instrument type variation will contribute to the uncertainty. Such measurement entity performance characteristics for a certain laboratory are easiest analyzed in comparison with similar measurements from other laboratories. This is, at least in the single-parametric model case typically solved by proficiency testing.

Proficiency testing for multiple-parameter models becomes more complex and most laboratories have not enough available resources to handle extensive control sample managing and collaboration with other laboratories. This approach is also complicated if the different laboratories belong to competing health-care groups. One solution would therefore be to have an independent party providing statistical and analytical support, but that is not involved in any actual evaluations of single subject measurements. Such a central party can establish contacts with many different local laboratories and may have access to many control sample measurements from a plurality of laboratories. Differences between different laboratories may be distinguished, assisting in the creation of individual measurement entity performance characteristics for the different laboratories. Furthermore, the total amount of available measurements will then also assist in evaluating the group effects of the model as well. An organization having two levels of actors is therefore proposed.

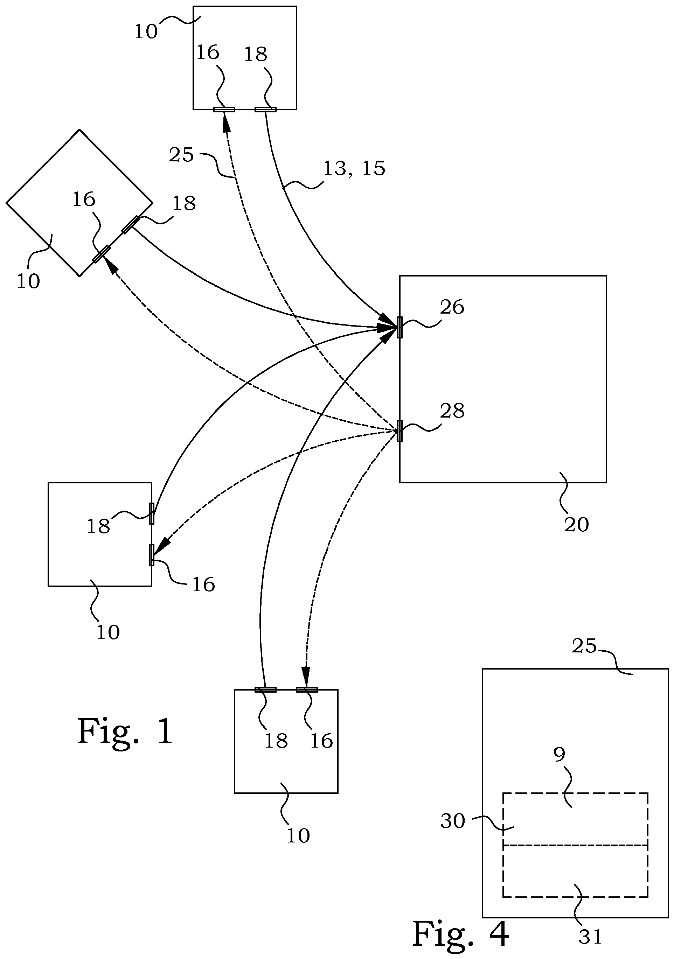

Figure 1 illustrates schematically the two-level organization. A system for support in medical conclusions 20 acts as a central party. The system for support in medical conclusions 20 has an input 26 configured for receiving different measurement data 15 of control samples, as well as for receiving different requests 13, which will be described further below. The system for support in medical conclusions 20 further comprises an output 28, configured for outputting models 25, also discussed more in details further below. A plurality of systems for medical conclusions 10 are provided and connected to the system for support in medical conclusions 20. The systems for medical conclusions 10 are thus local systems, e.g. at a laboratory. The systems for in medical conclusions 10 each comprise an output 18 configured for sending the measurement data 15 of control samples and the requests 13. The systems for medical conclusions 10 each also comprise an input 18 configured for receiving models 25.

From this setup, it can be concluded that the system for support in medical conclusions 20 has access to multitudes of measurement data 15 of control samples, but no access to any measurement data of actual subject measurement data e.g. associated with individual patients. The measurement data 15 of control samples are not connected to any particular patient and are not used for any type of diagnosis, since the conditions related to the control samples are known and postulated beforehand. This "open" data is shared between the systems for medical conclusions 10 and the system for support in medical conclusions 20. The measurement data associated with the individual patients are at the contrary kept within each system for medical conclusions 10. There is thus no risk for spreading sensitive information to other parties within this setup.

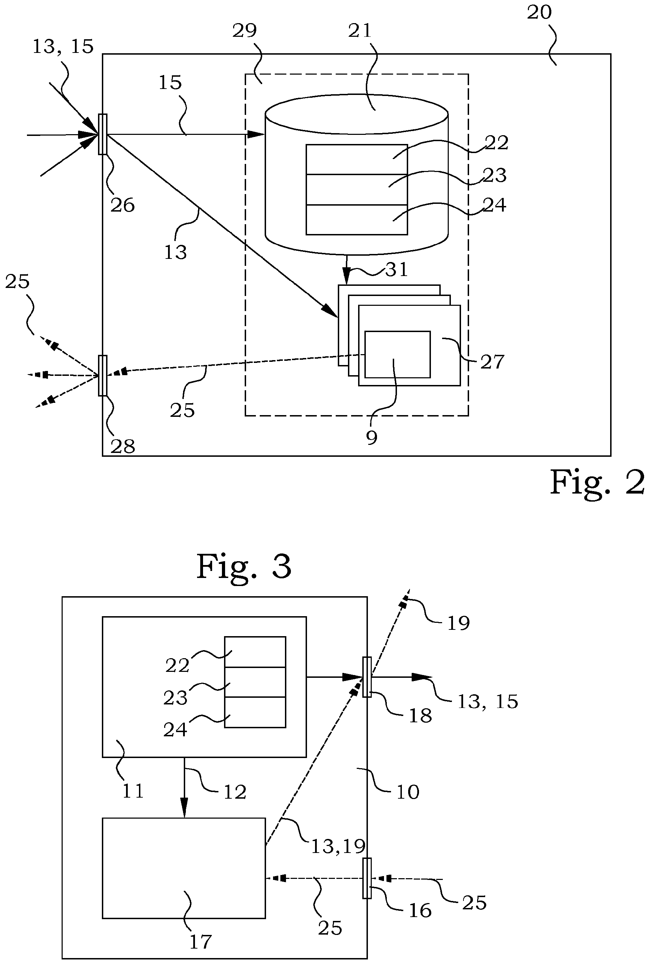

Figure 2 illustrates schematically an embodiment of system support in medical conclusions 20. The system for support in medical conclusions 20 comprises a processing system 29, having at least one processor 27 and an archive memory 21. The input 26 is configured for receiving measurement data 15 of control samples. The measurement data 15 of control samples comprise preferably measurement data 15 of control samples from more than one provider, thereby enabling proficiency testing. The measurement data 15 of control samples comprise multiple items of measured quantities 22 related to concentrations of at least three different biomarkers of at least two control samples, sample identity 23 of the at least two control samples, and measurement entity identification data 24 of the measuring entity that has performed the measurements. The processing system 29 is configured for storing the received measure quantities 22, the sample identity 23 and measurement entity identification data 24 in the archive memory 21.

The input 26 is further configured for receiving a request 13 from a requesting party. This request 13 can be a request for an adapted medical conclusion model associated with a first measurement entity. The processing system 29 is further configured for retrieving 31 stored data from the archive memory 21. This is made as a reply to the received request for an adapted medical conclusion model. The processing system 29, e.g. as performed in the processor(s) 27, is further configured for processing the retrieved 31 stored data, first into a certainty deduction model 9 associated with the first measurement entity. This certainty deduction model 9 comprises a group model and measurement entity performance characteristics. The group model is determined on stored data for control samples related to a predetermined set of multiple measurement entities. The measurement entity performance characteristics is determined on stored data for measurements of control samples related to the measurement entity that are performed less than a predetermined time ago. The processing system 29, e.g. as performed in the processor(s) 27, is further configured for processing the retrieved stored data into the requested adapted medical conclusion model 25. The requested adapted medical conclusion model is adapted to the previously created certainty deduction model 9 associated with the first measurement entity. The output 28 is configured for outputting models, in this case the adapted medical conclusion model 25 associated with the first measurement entity, to the requesting party.

Figure 3 illustrates schematically an embodiment of a system for medical conclusions 10. The system for medical conclusions 10 comprises a measurement entity 11. The measurement entity 11 is configured for measuring of quantities related to concentrations of at least three different biomarkers of samples.

The output 18 is configured for sending measurement data 15 of control samples to a system for support in medical conclusions. The measurement data 15 of control samples comprise multiple items of measured quantities 22 related to concentrations of at least three different biomarkers of at least two control samples, sample identity 23 of the at least two control samples, and measurement entity identification data 24 of the measuring entity. The output 18 is further configured for sending a request 13 to the system for support in medical conclusions, e.g. a request for an adapted medical conclusion model 25 associated with the measurement entity.

The input 16 is configured for receiving a model 25 from the system for support in medical conclusions, e.g. an adapted medical conclusion model 25 associated with the measurement entity. The adapted medical conclusion model is adapted based on a group model and measurement entity performance characteristics. As discussed above, the certainty deduction model comprises a group model and measurement entity performance characteristics. The group model is determined on stored data for control samples related to a predetermined set of multiple measurement entities. The measurement entity performance characteristics is determined on stored data for measurements of control samples related to the measurement entity that are performed less than a predetermined time ago.

The system for certainty estimation in medical conclusions 10 further comprises a processing unit 17. The processing unit 17 has access to measurements 12 of samples from the measurement entity 11. These measurements 12 of samples are made by the measurement entity 11 together with the at least two control samples. The processing unit 17 is configured deducing medical conclusions 19 from measurements 12 of said samples associated with individuals, made together with said at least two control samples. The provision of the medical conclusions 19 is performed by use of the received adapted medical conclusion model 25. The medical conclusions 19 are preferably outputted to any related party.

The adapted medical conclusion model 25 is based on a certainty deduction model 9 which in turn comprises, as indicated in Figure 4, a group model 30 and measurement entity performance characteristics 31. The group model 30 is determined on stored data from the archive for all control samples related to a predetermined set of multiple measurement entities. The measurement entity performance characteristics 31 is determined on stored data from the archive for measurements of control samples related to the first measurement entity that are performed less than a predetermined time ago. In this way, the certainty deduction model 9 can by the group model 30 make use of the collective knowledge about control sample measurements and thereby take advantage of the large available amount of data collected from all participating parties. At the same time, the certainty deduction model 9 can by the measurement entity performance characteristics 31 be tailored to each participating measurement entity, taking particular characteristics in measurement processes, time variations etc. into account. These properties are inherited by the adapted medical conclusion model 25.

The certainty estimate gives a measure of how reliable the conclusions drawn from the measurements are. If the measurements give a result that indicates that a certain condition is valid for a subject, the certainty estimate is a complement focusing on the reliability of the result. For instance, assume that an evaluation of a certain measurement indicates that a certain condition is present, but with a very small margin to e.g. some kind of used threshold. It would then be of great interest to know how reliable such results really are. May the actual indicated condition be an effect of measurement noise, so that a repeated measurement with a relatively high probability may show another result, or is the reliability of the result so high that it is possible to draw significant conclusions irrespective of the closeness to the threshold? Such situation may very well be the case, thanks to the improvements using different kinds of multi-parameter measurement evaluations. The certainty estimate may thus be an additional tool, besides the "normal" measurement results, that can be used for e.g. not only determine a "safe condition" and a "safe non-condition", but also an "unsecure situation". Appropriate continued handling of subjects associated with the measurements can then be performed. "Safe non-condition" subjects could safely be left without further treatment, "safe condition" subjects could immediately be given adequate treatment and for "unsecure situation" subjects, a continued sample measuring activity may be suitable. Such additional information may therefore save both medical resources as well as reducing the numbers of patients that have to suffer under difficult treatments.

The certainty estimate can also, as described further above, be a source of information useful for improving the medical conclusion model itself. The Model may e.g. be expanded into providing more than two conclusions. For instance, the medical conclusion model could be provided with three types of results; "safe condition", "safe non-condition", and "unsecure situation". By including this directly into the medical conclusion model, any person evaluating the results may not have to make the checks with certainty estimated in order to classify the results. In certain multiple-parameter models, uncertainties may also be used to classify the results further. The "unsecure situation" may for instance be divided in subgroups, depending on the magnitudes of the different contributions to the uncertainty. Such subgroups could e.g. be suggestions for certain follow-up measurements or treatments.

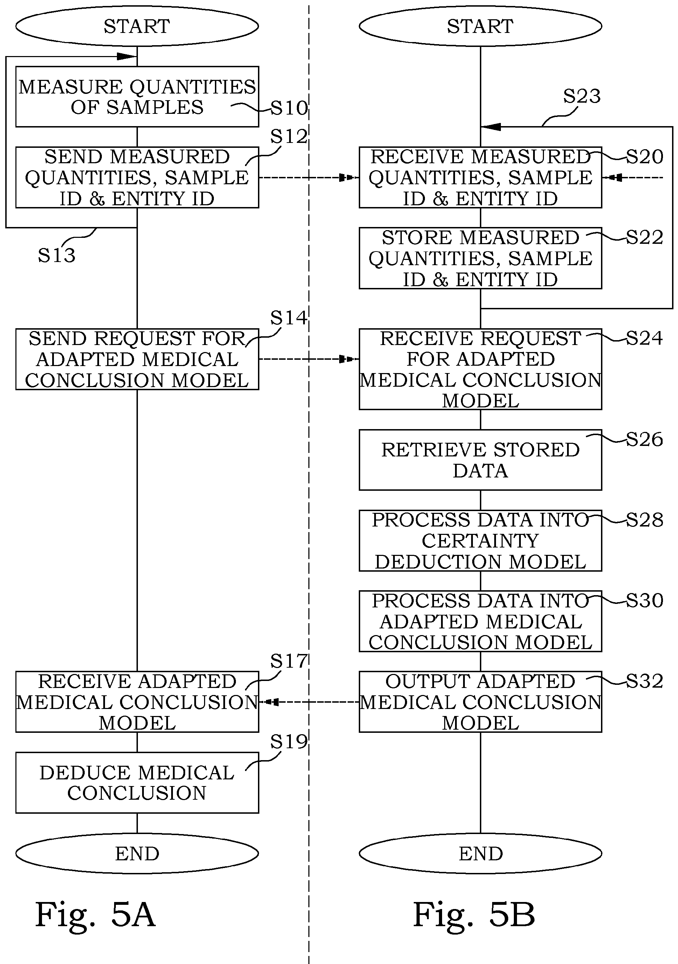

Figures 5A and 5B are flow diagrams of steps of embodiments of a method for providing non-diagnostic medical conclusions and a method for providing support in medical conclusions, respectively.

In step S10, quantities related to concentrations of at least three different biomarkers of samples are measured. The samples comprise a plurality of samples associated with individuals and at least two control samples. In step S12, data is sent to a system for support in medical conclusions. The sent data comprises measured quantities related to concentrations of at least three different biomarkers of the at least two control samples, sample identity of the at least two control samples, and measurement entity identification.

Steps S10 and S12 may be repeated one or several times, as indicated by the arrow S13.

In step S14, a request for an adapted medical conclusion model associated with the measurement entity is sent to the system for support in medical conclusions. Optionally, a request for an accompanying certainty deduction model is sent. In step S17, the adapted medical conclusion model associated with the measurement entity is received from the system for support in medical conclusions. If a certainty deduction model was requested in S14, it is also received in this step. The adapted medical conclusion model is adapted based on a certainty deduction model. The certainty deduction model comprises a group model and measurement entity performance characteristics. The group model is determined on stored data for control samples related to a predetermined set of multiple measurement entities. The measurement entity performance characteristics is determined on stored data for measurements of control samples related to the measurement entity that are performed less than a predetermined time ago. In step S19, medical conclusions are deduced from measurements of the samples associated with individuals, made together with the at least two control samples, based on the received adapted medical conclusion model and thus in dependence of the result from the certainty deduction model. The medical conclusions can in one embodiment be non-diagnostic.

These steps are performed by a local party being associated with the actual measurements and evaluation of the results. In a central party, adapted for supporting the local parties, the following steps may be performed.

In step S20, multiple items of data are received. This data comprises measured quantities related to concentrations of at least three different biomarkers of at least two control samples, sample identity of the at least two control samples, and measurement entity identification data of the measuring entity that has performed the measurements. The data can be received from the local party discussed here above, and/or from other local parties, as indicated by the two dotted arrows pointing to step S20. In other words, the step of receiving S20 multiple items of the measured quantities, the sample identities and the measurement entity identification data comprises receiving multiple items of the measured quantities, the sample identities and the measurement entity identification data comprises from more than one provider. This enables performing of proficiency tests. In step S22, the received measure quantities, the sample identity and measurement entity identification data are stored in an archive memory.

Steps S20 and S22 may be repeated one or several times, as indicated by the arrow S23.

In step S24, a request for an adapted medical conclusion model associated with a first measurement entity is received from a requesting party. In step S26, stored data is retrieving from the archive memory. In step S28, the retrieved stored data is processed in a processing system into the certainty deduction model associated with the first measurement entity. The certainty deduction model comprises a group model and measurement entity performance characteristics. The group model is determined on stored data from the archive memory for all control samples related to a predetermined set of multiple measurement entities. The measurement entity performance characteristics is determined on stored data from the archive memory for measurements of control samples related to the first measurement entity that are performed less than a predetermined time ago. In other words, the archived measurement entity performance characteristics contains recent data, so as to accurately reflect the current performance characteristics in the process of generating the adapted medical conclusion model.

In step S30, the retrieved stored data is processed into the adapted medical conclusion model, adapted to the certainty deduction model associated with the first measurement entity. The certainty deduction model is thereby utilized for identifying parts in the medical conclusion model where uncertainties in measurements may considerably influence the final conclusion. These uncertainty dependencies are furthermore specific for the requesting party by the use of the measurement entity performance characteristics. At the same time, the group model assures that proficiency testing aspects are taken into account.

In step S32, the adapted medical conclusion model associated with the first measurement entity is outputted to the requesting party.

The procedure can be further understood by describing an example. In a laboratory L, procedures to characterize samples for a multiparametric test are available. First, M subject samples and N control samples are subjected to measurement of intended biomarkers X, Y, Z. The resulting values for X, Y, Z in the N control samples are then submitted to a central server which is typically a different organization than laboratory L. The N control samples are of known identity and with known concentration profile of X, Y, Z, at least to the central server. The central server archives the resulting values for the submitted control samples in a database, possibly from many different laboratories. Next, the laboratory is provided access to an adapted medical conclusion model tailored for the laboratory, and optionally a certainty deduction model that is designed of estimating the reliability of the risk estimates made by the laboratory. The creation of the models will require input from the database with archived control sample data.

By use of the certainty deduction model, the reliability of any support estimate can be examined. With this input, the model for providing the medical conclusion can be improved to not only take the actual estimate of the medical conclusion into account but also the trustworthiness of the conclusion estimate. For instance, the adapted medical conclusion model may not only give a risk/non-risk output, but instead a more nuanced result, separating subjects of clearly no risk at all and subjects of high risk from subjects where e.g. continued examinations or measurements are advisable for achieving a trustworthy final conclusion.

Now, for each of the M subject samples, measurement results are extracted and a result which provides a support estimate for a medical conclusion is made according to the adapted medical conclusion model.

The total process represents a centralized and controlled procedure for estimating quality in a multiparametric setting. The fact that control sample values are delivered from the laboratory L to the central server for archiving means that the estimates of reliability can access historic data from one or more control samples and use that information to tailor the reliability analysis using the actual performance characteristic of the laboratory in question.

In one embodiment, the process of deducing the reliability model includes proficiency testing. Since the organization harbouring the central server may interact with multiple laboratories, and since different laboratories may use the same set of control samples, it is possible to compare control sample performance between laboratories. This is very similar to proficiency testing, albeit occurring in a more frequent manner. With the overview made available through the archiving of control sample data from multiple laboratories, and assuming that the same set of control samples are used in multiple laboratories, it becomes possible to pinpoint an error in either a laboratory, or in a control sample specimen, quickly. From a single laboratory point of view, it would be difficult or even impossible to achieve such a wide scale quality control.

It is beneficial to use three different control samples that are made available to multiple laboratories and for which the concentration values have been established using the same measurement procedure as was used when developing the risk algorithm. With three control samples, it becomes possible to cover low, medium and high level of each participating biomarker. It is not necessary to have one control sample with only low values, one with only medium and one with only high values. It is not necessary to limit the number of control samples to three, because in some cases four, five, six or even more control samples may be beneficial. It is not necessary to measure all control samples at the same time. It is possible, where the number of samples is large, to take turns and measure one control sample in the morning, another in the afternoon, and a third in the evening as a non-limiting example. Using two control samples, i.e. allowing a low and a high value of each biomarker, would also work with the method disclosed in this document.

The certainty deduction model will need input related to measurement performance characteristics, preferably both expected characteristics, e.g. specification of measurement procedure, and actual characteristics from laboratories in general or from a particular laboratory. Useful performance characteristics include, but are not limited, to the following:

Overall precision for each protein biomarker is useful. This could be expressed as the coefficient of variation, i.e. the standard deviation divided by the average, expressed in percent. Knowledge of precision for each measured protein biomarker means that the effect of stochastic error on the estimation of the risk can be estimated.

Stratified precision for concentration category of each biomarker, such as precision for low-range samples, precision for mid-range samples and precision for high-range samples can also be used. It is not unusual that the precision of a measurement procedure is different for low values and high values. Knowledge about how expected precision depends on a particular biomarker concentration is useful to estimate the effect of stochastic error on the risk algorithm output with greater precision.

The accuracy, also known as trueness, of each measurement procedure may also be important, at least in some applications. When a measurement procedure is inaccurate, a systematic shift of results is expected, and the impact of such a shift on the risk algorithm output is beneficial to have knowledge about.

Temporal stability of measurement procedures may influence the result. It is not uncommon that measurement procedures change over time. This can be due to seasonal variations, e.g. due to change in humidity or average room temperature, assay reagent stability issues, analytical instrument stability issues, and any other longer-term issues that may occur in a typical laboratory. Lack of temporal stability usually results in drift of expected results. Temporal stability is usually evaluated over a period of 1-2 weeks to 1-2 years, most often over a period of a few months. Knowledge of drift patterns and the similar is useful to estimate the effect of temporal stability on the risk algorithm output with greater precision.

Statistical distribution of repeated measurements during a predefined time-frame may be of use. With frequent, repeated measurements of control samples it becomes possible to estimate the distribution that the set of repeated measurements represent. The usual assumption is that repeated measurements conform to the normal distribution, but this is not necessarily a valid assumption. By registering and following the actual distribution of repeated measurements over time, important information about temporal stability and effect of changes, e.g. change of reagent batch, change of operator, and the similar, can be evaluated. This can in turn be translated to an estimated effect on risk estimation output.

One non-limiting method for estimating the effect on an estimation risk model output is to calculate the probability of that defects in input data changes the estimation risk model output to a level that the risk output crosses a predefined threshold. Many risk estimation models are applied in a manner where a value below a predefined threshold is considered low risk, and a value above the threshold is considered elevated risk. If input data results in a risk estimation model output which is close to the threshold, stochastic errors and other defects may have an impact big enough for a repeated measurement of the same sample specimen to obtain a risk algorithm value on the opposite side of the threshold, i.e. resulting in a different statement of the test. When there is access to performance characteristics of a particular laboratory, it is possible to calculate the probability of the risk algorithm providing a different result, in the light of being above or below a predefined threshold, upon remeasuring the sample specimen. If the probability is non-negligible, which for example could be greater than 1% (or 2%, or 3%, or 4%, or 5%, or 10%, or 25%, or 50%) chance of providing a different result, this fact can be conveyed to the health care provider so as to bring more complete information about the test result as such.

The benefit of using the sensitivity of the risk estimation model to input data defects is that the quality requirements on input data are estimated using the composite results as provided through the multiparametric risk estimation model. Hence, sensible quality characteristic requirements for each individual input data can be formulated in a manner that maximizes pass rate without compromising the quality of the results provided by the multiparametric risk estimation model.

When analysing biomarkers, it is preferred to have as diversative measurement outcomes as possible. In other words, a measurement that only gives an output of occurrence/non-occurrence of a biomarker is less useful in a following analysis that measurement giving e.g. a measured concentration value. A measured concentration value not only gives information about occurrence, but gives also information about the "strength" of the occurrence. An actual measurement value may therefore be very useful in e.g. multiparametric models. In order to reduce the data amount that is to be communicated between the measuring entity and an evaluation entity, the measured quantities may be associated to different predefined ranges, whereby only the range identity has to be communicated rather than the exact measure. Such a predefined range is preferably a predefined biologically relevant range. In such a way, the reduction of pure measurement data into categorized ranges does not necessarily reduce the usefulness of the information for multiparametric models. In particular, it is preferred if the the predefined biologically relevant ranges can be defined as concentration categories.

If predefined biologically relevant ranges, and in particular in the form of concentration categories, are used for representing the measurement data, it is also preferred if the control samples are selected accordingly. In other words, in one embodiment, at least two biomarkers of at least one of the control samples have known concentrations belonging to different concentration categories.

Furthermore, the output of the analysis of the adapted multiparametric model may also result in more than a "binary" result of existing/non-existing. As was discussed above, the uncertainty of the multiparametric model may be such that a different actual state would not be completely unlikely despite the fact that the result is under or over a certain threshold. This problem is, at least partly, solved by applying features within the multiparametric model itself. If the outcome from the multiparametric model is divided in three or more risk profiles, more information may be communicated to the person that will use the decision support. For instance, if the outcome of e.g. an analysis of a risk for relapse into a certain disease is divided into the profiles "none/low", "medium" and "high", different follow-up routines may be applied to these different groups, thereby tailoring suitable health resources to the actual estimated need. If such an approach is used, it is also preferred each of the control samples represents a predefined risk profile.

In one embodiment, the input of the system for certainty estimation support in medical conclusions is configured for receiving multiple items of measured quantities of concentrations of at least three different biomarkers of at least three control samples and sample identity of the at least three control samples and wherein the predefined risk profile comprises at least three levels.

For the system for medical conclusions, the measurement entity is in one embodiment configured for measuring the quantities related to concentrations of at least one of the biomarkers as a concentration value. Preferably, the measurement entity is configured for measuring the quantities related to concentrations of at least one of the biomarkers as an association to a predefined biologically relevant range. Preferably, the predefined biologically relevant ranges are concentration categories. At least two biomarkers of at least one of the control samples have known concentrations belonging to different concentration categories.

In one embodiment, each of the control samples represents a predefined risk profile.

In cases where a control sample represents a predefined risk profile, it may be possible and advisable to subject the measured values obtained for a control sample to the full risk model calculation, hence treating the control sample like it is an actual sample donated by an individual. For some risk models, this process is not straight-forward. In cases where the risk model combines input from widely different sources, for example when combining three entities such as (a) a self-declared patient information such as age, (b) one or more measured concentration values such as the concentration of PSA and free PSA in a donated plasma sample, and (c) one or more genotype values such as the allele composition of a predefine single nucleotide polymorphism (SNP) as measured on a donated blood sample, application of a risk model to a control sample from only one measurement setting is impossible. In such cases, it is possible to complement measured results from one control sample with static surrogate values (such as the average age and the typical allele composition, to relate to the example above) and feed the risk model with a combination of measured values and static surrogate values. With this approach, the risk model would result in an output that reflects the artificial individual composed by a control sample value and static surrogates, and would over time provide information about the risk level variation caused by stochastic and systematic noise from the measurement platform used to determine values for the control sample.

In a particular embodiment, the output of the system for medical conclusions is configured for sending measured quantities related to concentrations of at least three different biomarkers of at least three control samples and sample identity of the at least three control samples. The predefined risk profile comprises three levels.

In a process view, in an embodiment of a method for providing support in medical conclusions, the measured quantities of at least one of the biomarkers is a measured concentration value. In one embodiment, the measured quantities of at least one of the biomarkers is an association to a predefined biologically relevant range. Preferably, the predefined biologically relevant ranges are concentration categories. Preferably, at least two biomarkers of at least one of the control samples have known concentrations belonging to different concentration categories.

In one embodiment, each of the control samples represents a predefined risk profile.

In one particular embodiment, the step of receiving multiple items of the measured quantities, the sample identities and the measurement entity identification data comprises receiving multiple items of measured quantities of concentrations of at least three different biomarkers of at least three control samples and sample identity of the at least three control samples, wherein the predefined risk profile comprises three levels.

Likewise, in an embodiment of a method for providing medical conclusions, the step of measuring quantities related to concentrations of at least three different biomarkers comprises measuring the quantities related to concentrations of at least one of the biomarkers as a concentration value. In one embodiment, the step of measuring quantities related to concentrations of at least three different biomarkers comprises measuring the quantities related to concentrations of at least one of the biomarkers as an association to a predefined biologically relevant range. Preferably, the predefined biologically relevant ranges are concentration categories. Preferably, at least two biomarkers of at least one of the control samples have known concentrations belonging to different concentration categories.

In one embodiment, each of the control samples represents a predefined risk profile.

In a particular embodiment, the step of sending comprises sending of measured quantities related to concentrations of at least three different biomarkers of at least three control samples and sample identity of the at least three control samples, and wherein the predefined risk profile comprises three levels.

In the above discussed scenario, it is assumed that the control samples and the subject samples are measured in conjunction with each other and that the results are communicated to be stored centrally in the archive memory within a short time period. However, if delays in reporting of control samples are present, there might be problems to distinguish which measurements to use for deducing e.g. the measurement entity performance characteristics. Time stamping of the measurement results would be beneficiary. Therefore, in a preferred embodiment, the measurement entity of the system for medical conclusions is configured for registering a measuring time for each control sample. The output is thereby further configured for sending an indication of a control sample measuring time for each control sample. In a preferred embodiment of the system for support in medical conclusions, the input is further configured for receiving an indication of a control sample measuring time for each control sample.

Analogously, in a process view, a preferred embodiment of a method for providing medical conclusions comprises the further steps of registering a measuring time for each control sample and sending an indication of a control sample measuring time for each control sample. A preferred embodiment of a method for providing support in medical conclusions comprises the further step of receiving an indication of a control sample measuring time for each control sample. In such a way, time-dependent relations are possible to track, even if the measuring time of the control sample measurements cannot be assumed to be close to the time of communication.

The handling of control samples may also be improved in order to achieve further reliable decision supports. By spreading the same control samples to a number of different actors, factors being dependent on the actual control sample may be distinguished from factors being mainly dependent on the facility on which the measurements were performed. It is e.g. preferred if at least one control sample bulk is available in a plurality of individual control samples. This gives increased possibilities to redundancy measurements that can be used for different purposes.

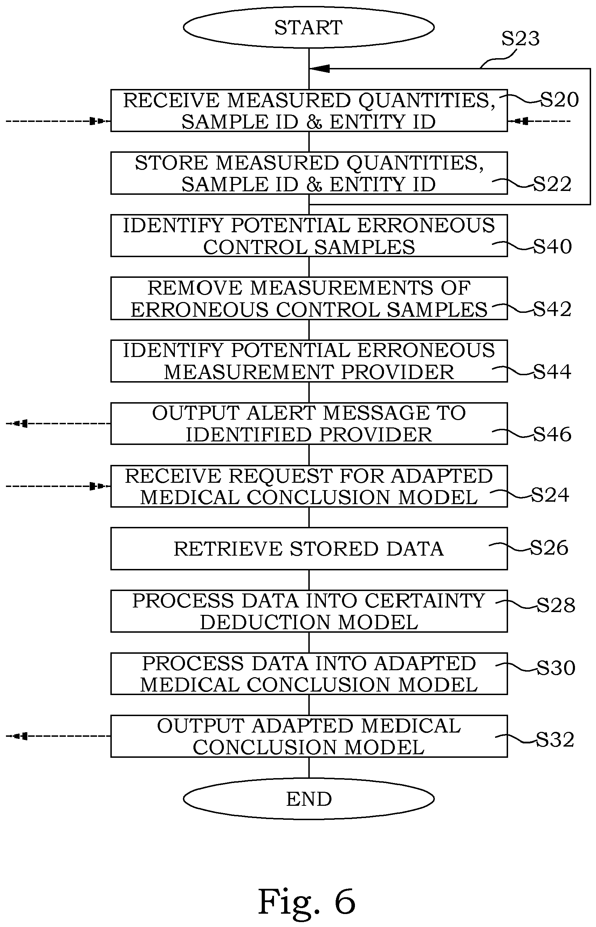

The correctness of the control samples is of importance. A control sample that in some manner has changed its characteristics or a control sample that in fact does not represent a concentration category it is assigned to may disturb the evaluation of certainty estimations. Since the central archive memory has access to multiple measurements on the same sample, statistical treatments of such data parts may be performed. It may therefore, as illustrated in step S40 of Figure 6, be possible to identifying a control sample that is associated with measurements falling outside an expected statistical variation, and to mark this control sample as a potential erroneous control sample. If this analysis is performed in a scientifically trustworthy manner, it is very likely that such a potential erroneous control sample may deteriorate the overall analysis in different degrees. It is then, as indicated in step S42, possible to remove these measurements associated with the identified erroneous control sample from the data stored in the central archive memory or at least exclude it to be incorporated in the analysis of the certainty estimation.

In other words, in one embodiment, the processing system of the system for support in medical conclusions is further configured for identifying a control sample that is associated with measurements falling outside an expected statistical variation as a potential erroneous control sample. The processing system is further configured to remove measurements associated with the identified erroneous control sample.

The use of control samples common for a plurality of laboratories also opens for a statistical treatment of laboratory-specific characteristics, i.e. the measurement entity performance characteristics.

In a preferred embodiment, the measurement entity performance characteristics comprises:

an overall precision for each biomarker,
a precision for each concentration category for each biomarker,
an accuracy for measured values for each biomarker in each control sample in relation to known concentrations,
a temporal stability of values submitted by a user identity during a predefined time frame, and/or
a distribution of measured values collected in during a predefined time frame for each biomarker in each control sample.

This information is as discussed above used for deducing the certainty deduction model, adapted to each individual laboratory. Strengths and weaknesses in the measurement equipment and/or routines are contributing to a certainty model that takes characteristics of each individual laboratory into account. These properties are the inherited by the adapted medical conclusion model.

The access to such data can also be used in order to support the individual laboratories in tracking possible errors. This is also illustrated in Figure 6. In one embodiment, the method for providing support in medical conclusions comprises the further step S44 of identifying a measurement provider that is associated with measurements falling outside an expected statistical variation as a potential error source. Furthermore, in step S46, an alert message can be outputted to the identified provider.

In other words, the processing system of the system for support in medical conclusions is further configured for identifying a measurement provider that is associated with measurements falling outside an expected statistical variation as a potential error source. The output is further configured for outputting an alert message to the identified provider.

The certainty deduction model, even if it is not directly connected to individual assignments of any medical conclusions, may nevertheless often have a certain impact of a finally made medical conclusion. As mentioned above, an uncertain result close to a threshold may be treated in a different way than a certain result at the same "position". For instance, of multiple risk profiles are defined, the uncertainty of the measurement analysis may be utilized to define the risk profile boundaries. It is thus according to the present ideas possible to use the uncertainty of the measurement analysis not only as a pure measure of the presently available medical conclusion model, but also as a means for improving the exactness of the medical conclusion model itself. Since the certainty deduction model is adapted to each individual measurement provider, there are possibilities also to adapt a medical conclusion model to each individual measurement provider, by incorporating adaptations based on the certainty deduction model and in particular the measurement entity performance characteristics.

The ideas presented above are best illustrated by showing an example of a system for certainty estimation in medical conclusions. In this example, the medical conclusion concerns testing for prostate cancer.

Blood samples, converted to plasma through centrifugation and supplemented with anticoagulant EDTA (ethylenediaminetetraacetic acid) from 384 individuals tested for prostate cancer using Stockholm3 were obtained. The Stockholm3 test is described in "Prostate cancer screening in men aged 50-69 years (STHLM3): a prospective population-based diagnostic study" as published in Lancet Oncology 2015 (http://dx.doi.org/10.1016/S1470-2045(15)00361-7). The Stockholm3 risk score was used as truth. All EDTA plasma samples were part of a technology transfer, meaning in addition to data from the regular Stockholm3 platform, new suppliers to a selection of assays were tested. This also means that each sample had its associated calculated Stockholm3 risk score, and the age of each individual was known. As part of the technology transfer process, samples from 264 individuals of the 384 were tested for plasma concentration of total prostate specific antigen (PSA), free PSA (i.e. the quantity of PSA which is not bound to other proteins) and growth differentiation factor 15 (GDF-15) using an enzyme linked immunosorbent assay (ELISA) in lab 1. Samples from 120 individuals of the 384 were tested for plasma concentration of PSA, free PSA and GDF-15 using ELISA in lab 2.

The same ELISA assays were used in both laboratories; total PSA (Diametra catalog number DKO137), free PSA (Diametra catalog number DKO138), GDF-15 concentration (Biovendor catalog number RD191135200R), and age (self reported). Estimated risk for prostate cancer in %, as calculated using Stockholm3, was available. Individuals with risk $\geq 11\%$ should be recommended to followed up.

Table 1. Performance characteristics of the three ELISA assays.

| | Precision (CV = standard deviation / average * 100%) Estimated using control sample QC52 | | |
| --- | --- | --- | --- |
| | Precision within run | Precision between run | Total precision |
| Lab1 total PSA | 4-10% | 4% | 7% |
| Lab1 free PSA | 2-7% | 4% | 7% |
| Lab1 GDF-15 | 3-8% | 4% | 9% |
| Lab2 total PSA | 1-5% | 7% | 4% |
| Lab2 free PSA | 5-16% | 16% | 10% |
| Lab2 GDF-15 | 1-12% | 3% | 9% |

A risk model to estimate cancer risk using ELISA assays was built in Lab 1 using the 264 data points. The risk model (RM1) became the following expression:

$$y = a1 * \sqrt{total\ PSA} + a2 * \sqrt{free\ PSA} + a3 * \frac{free\ PSA}{total\ PSA} + a4 *$$

$$\sqrt{(GDF - 15) * 0.001} + a5 * \frac{age-60}{10} + a6 \qquad (19)$$

with the accompanying parameter vector:

$$a1, ... a6 = [13.363 \quad -22.615 \quad 1.811 \quad 8.075 \quad 3.094 \quad -4.111] \quad (20)$$

Table 2. Concordance between laboratories.

| | Lab 1 Average QC52 | Lab2 Average QC52 | Lab1/Lab2 * 100% |
| --- | --- | --- | --- |
| Total PSA (ng/mL) | 4.6 | 4.4 | 105% |
| Free PSA (ng/mL) | 0.85 | 0.8 | 106% |
| GDF-15 (pg/mL) | 707 | 793 | 89% |

The risk model RM 1 displayed fair performance in predicting individuals with low risk. 94% of model statements of low risk were actually low Stockholm3 risk. However, the performance for indicating high risk was poor. 39% of model statements of high risk were actually high Stockholm3 risk. The model performance per se is, however, entirely outside the scope of the present technology.

Now, Lab 1 measures new samples for the purpose of applying RM 1 to new patients. The new samples are subjected to expected variance within run and between run of 7-9 % CV, see Table 1 above. Given control sample performance, it is possible, by Monte Carlo methods, to estimate the risk for an individual being sufficiently close to threshold so that measurement error can cause risk for a different statement than the one obtained for the actual values measured. Such a Monte Carlo assessment could comprise the following:

For each sample:

First use the set of input data, apply input data to the model, and achieve a model output.

Next, locate performance indicators for the input data that relate to a measurement procedure.
Next, take the set of input data, add randomly generated noise to each input data according to corresponding performance indicator.
Repeat the step of adding randomly generated noise a large number of times (such as 10 or 100 or 1000 or even bigger numbers).
Calculate the maximum change of model output that occurs with a predefined probability (for example 20%) due to addition of randomly generated noise.
Present the model output together with the confidence range to the user.

When applying the performance characteristics obtained at Lab 1 (CV 7%; 7%; 9% for total PSA, free PSA and GDF-15 respectively) and adding random noise according to the performance characteristics 1000 times, about 19% of the 264 samples were close enough to the threshold to accidentally end up on the other side with greater than 20% probability. When applying the performance characteristics obtained at Lab2 (CV 4%; 10%; 9% for total PSA, free PSA and GDF-15 respectively) and adding random noise according to the performance characteristics 1000 times, about 15% of the 120 samples measured at Lab2 were close enough to the threshold to accidentally end up on the other side of the threshold value with greater than 20% probability.

This indicates that the reaction of the model is uneven. An improvement of the total PSA assay gave more power to the model output than the loss of performance for free PSA and GDF-15. This type of reaction to altered performance characteristic profiles is very difficult to comprehend for an individual. It also indicates that the performance characteristic profile taken from the laboratory where the risk model was designed does not need to match the performance characteristic profile of the user laboratory. An individualized strategy for deducing certainty is beneficial to accurately describe the trueness of statements.

The performance characteristics of Lab2 was captured shortly after installation of equipment used in the laboratory. At a later point in time, the performance of Lab2 had improved to [3%; 5%; 3%] due to process optimization. Applying the performance characteristics of the optimized condition to the same 120 samples resulted in that about 11% of the 120 samples were close enough to the threshold to accidentally end up on the other side with greater than 20% probability. This indicates that also a seemingly small change in performance characteristics can alter the number of uncertain statements in a clear manner.

To reduce the burden of a laboratory to measure large quantities of control samples, the performance characteristics applied to the certainty deduction model can use multiple sources. One possible approach would be to rely on both the performance characteristics of the developing laboratory and combine that profile with the continuously measured performance characteristics of the user laboratory. A plausible method could be the following:

Define the performance characteristics to use for certainty deduction to be the average CV of (a) the development laboratory and (b) the actual user laboratory. In such a situation, one would rely on that a user laboratory is similar in terms of performance characteristics to the development laboratory.

In this particular example, this would mean that the Lab1 performance characteristics represent the developer and Lab2 performance characteristics represent the user. During initial operation the user laboratory would apply the average of [7 7 9] [4 10 9] which is [5.5 8.5 9]. After process optimization, the user laboratory would apply the average of [7 7 9] [3 5 3] which is [5 6 6]. Through combining one well characterized source, i.e. the developer laboratory, with one user source which may be less thoroughly characterized, a high level of reliability can be achieved.

The well characterized source which one assume the user laboratories are similar to may be described as a group model. The group model provides a basic expectation level of performance characteristics from which each user laboratory deviates from. Since it is possible to continuously compare user laboratory performance to the group model, it is also possible to verify if the assumption that the user laboratory is similar in terms of performance characteristics or not. Should a gross violation or repeated violation occur, it is possible to alert the user laboratory, or revert certainty deduction modelling to rely only on the user laboratory performance characteristics, or simply prevent the certainty deduction model from outputting a result.

There are also possibilities to improve also the medical conclusion support model to measurement entity performance characteristics of the measurement entity. After some time in operation using the setup defined here above, a new production batch of the measurement system for free PSA was delivered. It turned out that results from measurements using this new batch deviated from the previous batches, where the new batch produced values that in average were 30% higher. It is known that different batches normally differ to some extent. A systematic change of 30% is typically considered large but still borderline acceptable.

The risk model would now be exposed to two types of deviations; the stochastic deviation seen as "noise" and modelled here above, and a systematic deviation due to the change of batch for one measurement system: FPSA. One option to manage this situation would be to incorporate the systematic change as a measurement error and estimate the certainty of statements in view of a 30% systematic change on one of the input data. This would however lead to that almost half of the measure samples being classified as close enough to the threshold to accidentally end up on the other side of the threshold with greater than 20% probability.

Another, better, option is to allow the risk model to adapt to the confirmed batch change. If performance characteristics is amended with information about the control sample absolute values obtained in the measurement process, batch properties can be incorporated into the risk model to compensate for systematic deviations that stem from e.g. batch changes.

With this adaptable option, the risk model would accept input from performance characterization. This means that the performance characterization would need to be a continuous process that share data to a central repository from which current characteristics, in particular absolute values of known control samples, can be shared with the risk model. Upon the risk model having access to confirmed current values of known control samples, the risk model could be adjusted to compensate for any change in systematic values. In the particular case discussed in the present example, the risk model could for example process input values for the free PSA by multiplication with (1/ 1.30) so as to shift the input values from the deviant batch towards the average batch characteristics. After such a shift, the risk model can be applied and provide output that is consistent irrespective of batch behaviour.

$$\text{BAtotal PSA} = \text{BatchAdjustmentFactor1} * \text{total PSA} \qquad (21)$$

$$\text{BAfree PSA} = \text{BatchAdjustmentFactor2} * \text{free PSA} \qquad (22)$$

$$\text{BAGDF-15} = \text{BatchAdjustmentFactor3} * \text{GDF-15} \qquad (23)$$

$$y = a1 * \sqrt{BAtotal\ PSA} + a2 * \sqrt{BAfree\ PSA} + a3 * \frac{BAfree\ PSA}{BAtotal\ PSA} + a4 *$$

$$\sqrt{(BAGDF - 15) * 0.001} + a5 * \frac{age - 60}{10} + a6 \qquad (24)$$

with the accompanying parameter vector as in (20) above.

In this particular case of the new free PSA batch deviating 30%, the following Batch adjustment factors can be applied:

$$\text{BatchAdjustmentFactor1} = 1 \qquad (21A)$$

$$\text{BatchAdjustmentFactor2} = 1/1.30 \qquad (22A)$$

$$\text{BatchAdjustmentFactor3} = 1 \qquad (23A)$$

The determination of a suitable BatchAdjustment factor can be made across different laboratories, consistent with group modelling performance characteristics reasoning.

After adjusting for batch differences, the control samples will mimic a combination of stochastic variation, i.e. the precision characteristic in e.g. Table 1, and the systematic error of the new batch after batch adjustment. The batch adjustment process should be good enough to make the effects of batch changes a non-dominant error source.

By supplying such BatchAdjustment factor from a system for certainty estimation support in medical conclusions to a system for certainty estimation in medical conclusions, an adjusted and improved medical conclusion support model is provided. The medical conclusion support model is adapted to measurement entity performance characteristics of the measurement entity.

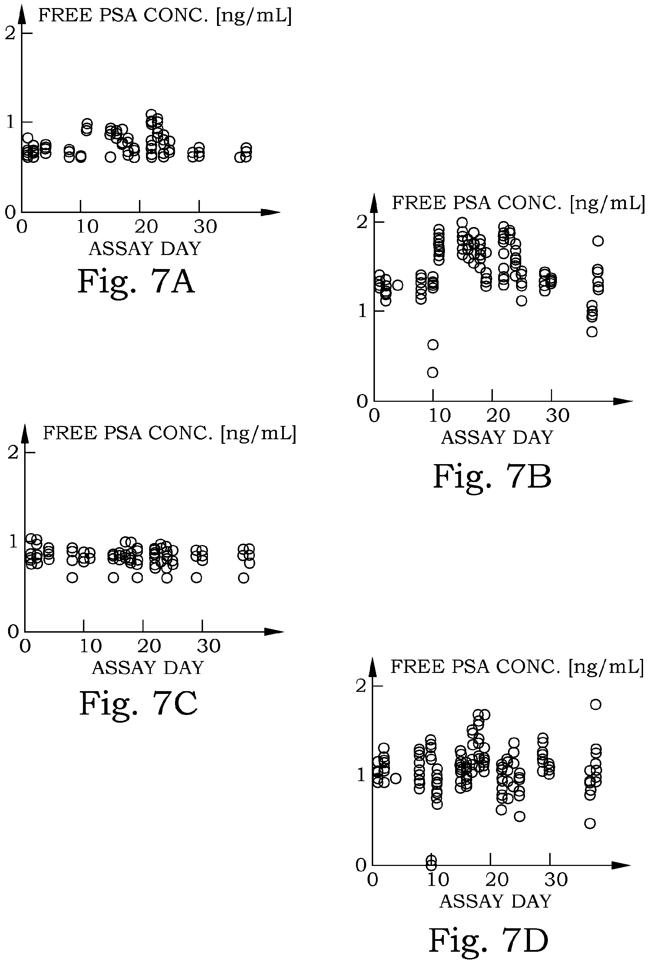

Batch adjustment was implemented in Lab2. QC52 was designated the control sample that defined the Batch-AdjustmentFactor and another control sample, QC5, was given the task of controlling that the BatchAdjustmentFactor was appropriate. Each assay was designed such that it included both QC52 and QC5 control samples. Based on results from initial operation, QC52 should have a value of 0.85 ng/ml and QC5 should have a free PSA value of 1.67 ng/mL. A while after having implemented BatchAdjustment, results from 20 assays obtained over 40 calendar days using three different batches was analyzed (Table 3, Figures 7A-D). Should one rely on non-BatchAdjusted data, the performance characteristics for precision would be in the order of 18-19%, with absolute values deviating about 10-16% from initial values. With BatchAdjustment implemented in the risk model RM1, the performance characteristics for precision was reduced to about 11% with absolute values deviating only 3-5%. This in turn means that the BatchAdjustment process recovers the initial performance characteristics and lead to fewer results being classified as uncertain by the certainty deduction model.

Table 3. Free PSA results obtained with two different lots.

| | Free PSA as measured | | Free PSA batchadjusted to QC52 | |
|---|---|---|---|---|
| | Average (ng/mL) | Precision (CV) | Average (ng/mL) | Precision (CV) |
| QC52 | 0.71 | 18% | 0.83 | 11% |
| QC5 | 1.51 | 19% | 1.74 | 11% |

In Figure 7A, free PSA concentration as measured for QC52 is illustrated. In Figure 7B, free PSA concentration as measured for QC5 is illustrated. In Figure 7C, batch-adjusted free PSA concentration for QC52 is illustrated. In Figure 7D, batch-adjusted free PSA concentration for QC5 is illustrated.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A system for support in medical conclusions (20), comprising:

     - a processing system (29), having at least one processor (27) and an archive memory (21);
     - an input (26) configured for receiving multiple items of:

          measured quantities (22) related to concentrations of at least three different biomarkers of at least two control samples;
          sample identity (23) of said at least two control samples; and
          measurement entity identification data (24) of the measuring entity that has performed the measurements;

     wherein said processing system (29) being configured for storing said received measure quantities (22), said sample identity (23) and measurement entity identification data (24) in said archive memory (21);
     - said input (26) being further configured for receiving a request (13) for an adapted medical conclusion model (25) associated with a first measurement entity from a requesting party;

   wherein said processing system (29) being further configured for retrieving stored data from said archive memory (21);
   wherein said processing system (29) being further configured for processing said retrieved stored data into a certainty deduction model (9) associated with said first measurement entity;
   wherein said certainty deduction model (9) comprises a group model (30) and measurement entity performance characteristics (31);
   wherein said group model (30) is determined on stored data from said archive for all control samples related to a predetermined set of multiple measurement entities;
   wherein said measurement entity performance characteristics (31) is determined on stored data from said archive for measurements of control samples related to said first measurement entity that are performed less than a predetermined time ago;
   wherein said processing system being further configured for processing said retrieved stored data into said adapted medical conclusion model (25), adapted to said certainty deduction model (9) associated with said first measurement entity; and

     - an output (28) configured for outputting said adapted medical conclusion model (25) associated with said first measurement entity to said requesting party.

2. The system according to claim 1, **characterized in that** said input (26) is configured for receiving said measured quantities (22), said sample identities (23) and said measurement entity identification data (24) from more than one provider, thereby enabling proficiency testing.

3. The system according to claim 1 or 2, **characterized in that** said measured quantities (22) of at least one of said biomarkers is a measured concentration value.

4. The system according to any of the claims 1 to 3, **characterized in that** said input (26) is further configured for receiving an indication of a control sample measuring time for each control sample.

5. The system according to any of the claims 1 to 4, **characterized in that** said measurement entity performance characteristics comprises at least one of:

> an overall precision for each biomarker;
> a precision for each concentration category for each biomarker;
> an accuracy for measured values for each biomarker in each control sample in relation to known concentrations;
> a temporal stability of values submitted by a user identity during a predefined time frame; and
> a distribution of measured values collected in during a predefined time frame for each biomarker in each control sample.

6. The system according to any of the claims 1 to 5, **characterized in that** said wherein said processing system (29) being further configured for identifying a measurement provider being associated with measurements falling outside an expected statistical variation as a potential error source; wherein said output (28) is further configured for outputting an alert message to said identified provider.

7. The system according to any of the claims 1 to 6, **characterized in that** said wherein said processing system (29) being further configured for identifying a control sample being associated with measurements falling outside an expected statistical variation as a potential erroneous control sample; wherein said processing system (29) being further configured to remove measurements associated with said identified erroneous control sample.

8. A system for medical conclusions (10), comprising:

> - a measurement entity (11) for measuring of quantities related to concentrations of at least three different biomarkers of samples;
> said samples comprising a plurality of samples associated with individuals and at least two control samples;
> - an output (18) configured for sending:

>> measured quantities (22) related to concentrations of at least three different biomarkers of said at least two control samples;
>> sample identity (23) of said at least two control samples; and
>> measurement entity identification (24),
>> to a system for support in medical conclusions;
>> wherein said output (18) being further configured for sending a request (13) for an adapted medical conclusion model (25) associated with said measurement entity to said system for support in medical conclusions (20);

> - an input (16) configured for receiving said adapted medical conclusion model (25) associated with said measurement entity from said system for support in medical conclusions (20);
> wherein said adapted medical conclusion model (25) is adapted based on a certainty deduction model (9) comprising a group model (30) and measurement entity performance characteristics (31);
> wherein said group model (30) is determined on stored data for control samples related to a predetermined set of multiple measurement entities;
> wherein said measurement entity performance characteristics (31) is determined on stored data for measurements of control samples related to said measurement entity that are performed less than a predetermined time ago; and
> - a processing unit (17) configured for deducing medical conclusions from measurements of said samples associated with individuals, made together with said at least two control samples, by use of said received adapted medical conclusion model (25).

9. The system according to claim 8, **characterized in that** said measurement entity (11) is configured for measuring said quantities related to concentrations of at least one of said biomarkers as a concentration value.

**10.** The system according to claim 8 or 9, **characterized in that** said measurement entity (11) is configured for registering a measuring time for each control sample, wherein said output (18) is further configured for sending an indication of a control sample measuring time for each control sample.

**11.** A method for providing support in medical conclusions, comprising the steps of:

- receiving (S20) multiple items of:

measured quantities (22) related to concentrations of at least three different biomarkers of at least two control samples;
sample identity (23) of said at least two control samples; and
measurement entity identification data (24) of the measuring entity that has performed the measurements;

- storing (S22) said received measure quantities (22), said sample identity (23) and measurement entity identification data (24) in an archive memory (21);
- receiving (S24), from a requesting party, a request (13) for an adapted medical conclusion model (25) associated with a first measurement entity;
- retrieving (S26) stored data from said archive memory (21);
- processing (S28), in a processing system (29), said retrieved stored data into a certainty deduction model (9) associated with said first measurement entity;
wherein said certainty deduction model (9) comprises a group model (30) and measurement entity performance characteristics (31);
wherein said group model (30) is determined on stored data from said archive memory (21) for all control samples related to a predetermined set of multiple measurement entities;
wherein said measurement entity performance characteristics (31) is determined on stored data from said archive memory (21) for measurements of control samples related to said first measurement entity that are performed less than a predetermined time ago;
- processing (S30) said retrieved stored data into said adapted medical conclusion model (25), adapted to said certainty deduction model (9) associated with said first measurement entity; and
- outputting (S32) said adapted medical conclusion model (25) associated with said first measurement entity to said requesting party.

**12.** The method according to claim 11, **characterized in that** said step of receiving multiple items of said measured quantities, said sample identities and said measurement entity identification data comprises receiving multiple items of said measured quantities, said sample identities and said measurement entity identification data from more than one provider, wherein the method comprises the further step of performing proficiency tests.

**13.** The method according to claim 11 or 12, **characterized in that** said measured quantities (22) of at least one of said biomarkers is a measured concentration value.

**14.** The method according to any of the claims 11 to 13, **characterized by** the further step of receiving an indication of a control sample measuring time for each control sample.

**15.** A method for providing non-diagnostic medical conclusions, comprising the steps of:

- measuring (S10) quantities related to concentrations of at least three different biomarkers of at least two control samples;
said samples comprising a plurality of samples associated with individuals and at least two control samples;
- sending (S12):

measured quantities (22) related to concentrations of at least three different biomarkers of said at least two control samples;
sample identity (23) of said at least two control samples; and
measurement entity identification (24),
to a system for support in medical conclusions (20);

- sending (S14) a request for an adapted medical conclusion model (25) associated with said measurement entity to said system for support in medical conclusions (20);

**EP 4 195 212 A1**

- receiving (S17) said adapted medical conclusion model (25) associated with said measurement entity from said system for support in medical conclusions (20);

wherein said adapted medical conclusion model (25) is adapted based on a certainty deduction model (9) comprising a group model (30) and measurement entity performance characteristics (31);

wherein said group model (30) is determined on stored data from said archive for all control samples related to a predetermined set of multiple measurement entities;

wherein said measurement entity performance characteristics (31) is determined on stored data from said archive for measurements of control samples related to said measurement entity that are performed less than a predetermined time ago; and

- deducing (S19) non-diagnostic medical conclusions from measurements of said samples associated with individuals, made together with said at least two control samples, based on said received adapted medical conclusion model (25).

**19**

10

16    18

25

13, 15

16    18

10

26

28

18

20

16

10

Fig. 1

18    16

10

25

9

30

31

Fig. 4

Fig. 2

Fig. 3

START

MEASURE QUANTITIES
OF SAMPLES ~S10

SEND MEASURED
QUANTITIES, SAMPLE
ID & ENTITY ID ~S12

S13

SEND REQUEST FOR
ADAPTED MEDICAL
CONCLUSION MODEL ~S14

RECEIVE ADAPTED
MEDICAL CONCLUSION
MODEL ~S17

DEDUCE MEDICAL
CONCLUSION ~S19

END

Fig. 5A

START

S23

RECEIVE MEASURED
QUANTITIES, SAMPLE
ID & ENTITY ID ~S20

STORE MEASURED
QUANTITIES, SAMPLE
ID & ENTITY ID ~S22

RECEIVE REQUEST
FOR ADAPTED
MEDICAL CONCLUSION
MODEL ~S24

RETRIEVE STORED
DATA ~S26

PROCESS DATA INTO
CERTAINTY
DEDUCTION MODEL ~S28

PROCESS DATA INTO
ADAPTED MEDICAL
CONCLUSION MODEL ~S30

OUTPUT ADAPTED
MEDICAL CONCLUSION
MODEL ~S32

END

Fig. 5B

```
                    ┌──────────────────┐
                    │      START       │
                    └──────────────────┘                    S23
                              │
                              ▼◄──────────────────────────────┐
              ┌───────────────────────────────────┐           │
    ─ ─ ─ ─►► │ RECEIVE MEASURED QUANTITIES,      │~S20   ◄─ ─│
              │     SAMPLE ID & ENTITY ID          │           │
              ├───────────────────────────────────┤           │
              │ STORE MEASURED QUANTITIES,        │           │
              │     SAMPLE ID & ENTITY ID          │~S22       │
              └───────────────────────────────────┘           │
                              │
              ┌───────────────────────────────────┐
              │ IDENTIFY POTENTIAL ERRONEOUS       │
              │     CONTROL SAMPLES      │~S40
              └───────────────────────────────────┘
                              │
              ┌───────────────────────────────────┐
              │ REMOVE MEASUREMENTS OF             │
              │ ERRONEOUS CONTROL SAMPLES │~S42
              └───────────────────────────────────┘
                              │
              ┌───────────────────────────────────┐
              │ IDENTIFY POTENTIAL ERRONEOUS       │
              │ MEASUREMENT PROVIDER      │~S44
              └───────────────────────────────────┘
                              │
              ┌───────────────────────────────────┐
    ◄─ ─ ─ ─  │ OUTPUT ALERT MESSAGE TO           │
              │ IDENTIFIED PROVIDER      │~S46
              └───────────────────────────────────┘
                              │
              ┌───────────────────────────────────┐
    ─ ─ ─ ─►► │ RECEIVE REQUEST FOR ADAPTED        │
              │ MEDICAL CONCLUSION MODEL  │~S24
              └───────────────────────────────────┘
                              │
              ┌───────────────────────────────────┐
              │     RETRIEVE STORED DATA  │~S26
              └───────────────────────────────────┘
                              │
              ┌───────────────────────────────────┐
              │ PROCESS DATA INTO CERTAINTY │~S28
              │ DEDUCTION MODEL                    │
              └───────────────────────────────────┘
                              │
              ┌───────────────────────────────────┐
              │ PROCESS DATA INTO ADAPTED │~S30
              │ MEDICAL CONCLUSION MODEL           │
              └───────────────────────────────────┘
                              │
              ┌───────────────────────────────────┐
    ◄─ ─ ─ ─  │ OUTPUT ADAPTED MEDICAL    │~S32
              │ CONCLUSION MODEL                   │
              └───────────────────────────────────┘
                              │
                              ▼
                    ┌──────────────────┐
                    │       END        │
                    └──────────────────┘
```

# Fig. 6

Fig. 7A

Fig. 7B

Fig. 7C

Fig. 7D

Europäisches
Patentamt
European
Patent Office
Office européen
des brevets

# EUROPEAN SEARCH REPORT

Application Number

EP 21 21 3167

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/114559 A1 (YAMAGUCHI TADAYUKI [JP] ET AL) 15 May 2008 (2008-05-15) <br> * paragraph [0026] – paragraph [0027] * <br> * paragraph [0038] * <br> * paragraph [0044] * <br> * paragraph [0047] – paragraph [0051] * <br> * paragraph [0053] * <br> * paragraph [0056] * <br> * paragraph [0067] * <br> * paragraph [0070] – paragraph [0071] * <br> * paragraph [0083] * <br> * figures 1-6 * <br> ----- | 1-15 | INV. <br> G16H10/40 <br> G16H50/20 |
| A | US 2016/350477 A1 (MCHUGH LEO CHARLES [US]) 1 December 2016 (2016-12-01) <br> * paragraph [0094] – paragraph [0224] * <br> * figures 1-10 * <br> ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 25 May 2022 | Hauber, Jörg |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 21 3167

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2022

| Patent document cited in search report | | Publication date | Patent family member(s) | | Publication date |
|---|---|---|---|---|---|
| US 2008114559 | A1 | 15-05-2008 | CN | 101149618 A | 26-03-2008 |
| | | | EP | 1903461 A2 | 26-03-2008 |
| | | | JP | 4817251 B2 | 16-11-2011 |
| | | | JP | 2008076267 A | 03-04-2008 |
| | | | US | 2008114559 A1 | 15-05-2008 |
| US 2016350477 | A1 | 01-12-2016 | AU | 2016267392 A1 | 07-12-2017 |
| | | | CA | 2986787 A1 | 01-12-2016 |
| | | | CN | 108323184 A | 24-07-2018 |
| | | | EP | 3304093 A1 | 11-04-2018 |
| | | | HK | 1249182 A1 | 26-10-2018 |
| | | | JP | 2018525703 A | 06-09-2018 |
| | | | US | 2016350477 A1 | 01-12-2016 |
| | | | US | 2018107783 A1 | 19-04-2018 |
| | | | WO | 2016187655 A1 | 01-12-2016 |

EPO FORM P0459

For more details about this annex : see Official Journal of the European Patent Office, No. 12/82